# EUROPÄISCHE PATENTSCHRIFT

(11) **EP 4 003 312 B1**
(45) Veröffentlichungstag und Bekanntmachung des Hinweises auf die Patenterteilung: **12.07.2023**
(21) Anmeldenummer: 20751097.5
(22) Anmeldetag: 28.07.2020
(51) Int. Cl.: A61K 31/137, A61K 9/70

(54) **VERFAHREN ZUR HERSTELLUNG VON HAFTKLEBEMASSEN ZUR VERWENDUNG IN EINEM TRANSDERMALEN THERAPEUTISCHEN SYSTEM**
PROCESS FOR PREPARING ADHESIVE COMPOUNDS FOR USE IN A TRANSDERMAL THERAPEUTIC SYSTEM
PROCÉDÉ DE PRÉPARATION DE COMPOSÉS ADHÉSIFS DESTINÉS À ÊTRE UTILISÉS DANS UN SYSTÈME THÉRAPEUTIQUE TRANSDERMIQUE

(30) Priorität: 29.07.2019 DE 102019120403
(43) Veröffentlichungstag der Anmeldung: 01.06.2022
(73) Patentinhaber: LTS LOHMANN Therapie-Systeme AG, 56626 Andernach (DE)
(72) Erfinder: ORTH, Michael, 53604 Bad Honnef (DE); TELLKAMP-SCHEHR, Tobias, 40882 Ratingen (DE); HAMMES, Florian, 56626 Andernach (DE); LODDER-GADACZEK, Julia, 65618 Selters Taunus (DE)
(74) Vertreter: Davepon, Björn
(86) Internationale Anmeldenummer: PCT/EP2020/071312
(87) Internationale Veröffentlichungsnummer: WO 2021/018917

(56) Entgegenhaltungen:
- EP-A2- 0 887 075
- WO-A1-95/18603
- WO-A1-2019/079291
- Henkel Ltd: "DURO-TAK and GELVA Transdermal Pressure Sensitive Adhesives DURO-TAK and GELVA Transdermal Pressure Sensitive Adhesives PRODUCT SELECTION GUIDE", , 20. November 2013 (2013-11-20), XP055141577, Gefunden im Internet: URL:http://www.henkelna.com/us/content_dat a/330922_11061_LT5343_Product_selector2_We b863600.pdf [gefunden am 2014-09-19]

## Beschreibung

Die vorliegende Erfindung betrifft ein Verfahren zur Herstellung einer wirkstoffhaltigen Klebstoffformulierung zur Verwendung in einem transdermalen therapeutischen System sowie ein transdermales therapeutisches System enthaltend diese wirkstoffhaltige Klebstoffformulierung, wie durch die Ansprüche definiert. Die Erfindung betrifft ferner die Verwendung des transdermalen therapeutischen Systems und ein Kit, das dieses transdermale therapeutische System enthält, wie durch die Ansprüche definiert.

Bei transdermalen therapeutischen Systemen (im Folgenden kurz "TTS") handelt es sich um flächenförmige, schichtweise aufgebaute pharmazeutische Produkte, bei denen mindestens ein pharmakologisch aktiver Wirkstoff mit oder ohne Hilfsstoffe (z.B. Penetrationsbeschleuniger) in eine ggf. haftklebende Polymermatrix eingebettet ist. In der Regel wird diese Polymermatrix hergestellt, indem eine Trägerfolie mit der den Wirkstoff enthaltende Polymermasse beschichtet und anschließend mit einer Abdeckfolie versehen wird, welche auch während der Applikation des transdermalen therapeutischen Systems auf der Haut verbleibt. Die Trägerfolie dient als Schutzschicht für die Polymermatrix während der Dauer der Lagerung und gegebenenfalls als Applikationshilfe für die spätere Anwendung des transdermalen therapeutischen Systems. Transdermale therapeutische Systeme ermöglichen eine kontinuierliche Wirkstoffzufuhr über den gesamten Applikationszeitraum. Sie sind daher bezüglich ihrer Konzentrations-Zeit-Profile vergleichbar mit Dauertropfinfusionen. Transdermale therapeutische Systeme werden üblicherweise bei medizinischen Indikationen eingesetzt, bei denen ein Wirkstoff über einen unbegrenzten oder zumindest einen längeren Zeitraum verabreicht werden muss, wie beispielsweise bei chronischen Krankheiten oder bei einer Hormonsubstitutionstherapie. Zahlreiche transdermale therapeutische Systeme mit unterschiedlichen Wirkstoffen oder Wirkstoffkombinationen befinden sich heute auf dem Arzneimittelmarkt.

Eine Reihe von kommerziell erhältlichen transdermalen therapeutischen Systemen ist als sogenannte Matrixsysteme konstruiert. Hierbei handelt es sich um Systeme, bei denen die haftklebend oder nicht-haftklebend ausgerüstete Polymermatrix den Wirkstoff in gelöster oder suspendierter Form enthält. Für die Verwendung auf der Haut ausgelobte Haftklebstoffe auf Acrylatbasis sind beispielsweise unter dem Markennamen DURO-TAK^{®} und GELVA^{®} (beide von der Henkel AG & Co. KGaA) erhältlich. Diese kommerziell erhältlichen Haftklebstoffe werden in Lösungsmittel gelöst vertrieben und im Rahmen von herkömmlichen Herstellungsprozessen zunächst mit zumindest einem Wirkstoff und evtl. Hilfs- und Zusatzstoffen vermischt, auf einen Träger aufgetragen und dann das Lösungsmittel entfernt, so dass eine haftklebende Polymermatrix auf dem Träger erhalten wird. Kommerziell erhältliche Haftklebstoffe enthalten in den meisten Fällen ein Carbonsäureester-basiertes Lösungsmittel insbesondere die unter dem Markennamen DURO-TAK^{®} und GELVA^{®} erhältlichen Haftklebstoffe auf Acrylatbasis. Darüber hinaus enthalten kommerziell erhältliche Haftklebestoffe auch bei der Synthese der Polymermatrix nicht umgesetzte Monomere. Die unter dem Markennamen DURO-TAK^{®} und GELVA^{®} erhältlichen Haftklebstoffe enthalten in erste Linie das Vinylacetat als ein solches nicht umgesetztes Monomer.

Es wurde jedoch festgestellt, dass bei der Verwendung von derartigen kommerziell erhältlichen Haftklebstoffen für die Herstellung von transdermalen therapeutischen Systemen ein Teil des Wirkstoffs, der zur Herstellung des transdermalen therapeutischen Systems zu dem Haftklebstoff gegeben wurde, bereits kurz nach Beendigung des Herstellungsprozesses nicht mehr in der Polymermatrix nachweisbar ist. Außerdem wurden bereits kurz nach dem Herstellungsprozess Reaktionsprodukte in der Polymermatrix nachgewiesen, die sehr wahrscheinlich aufgrund von Reaktionen und/oder Wechselwirkungen zwischen dem pharmakologisch aktiven Wirkstoff und der Polymermatrix und/oder darin enthaltenen Lösungsmitteln und/oder Monomeren aus der Polymermatrix des Haftklebestoffes entstanden sind.

Da zum Teil sehr teure Wirkstoffe in transdermalen therapeutischen Systemen eingesetzt werden, ist die verringerte Verfügbarkeit des pharmakologisch aktiven Wirkstoffs in transdermalen therapeutischen Systemen bei Herstellungsverfahren, bei denen kommerziell erhältlichen Haftklebstoffe eingesetzt werden, unwirtschaftlich. Des Weiteren ist es bei transdermalen therapeutischen Systeme, die über einen längeren Zeitraum auf der Haut verbleiben sollen, notwendig, dass diese genügend Wirkstoff aufweisen, um sicherzustellen, dass über die gesamte Anwendungsdauer auch gegen Ende des Anwendungszeitraums die therapeutisch notwendige Menge an Wirkstoff abgegeben wird. Des Weiteren verringern Reaktionen und/oder Wechselwirkungen zwischen dem pharmakologisch aktiven Wirkstoff und der Polymermatrix und/oder darin enthaltenen Lösungsmitteln die Lagerbeständigkeit des transdermalen therapeutischen Systems, d.h. nach einer Lagerung ist weniger Wirkstoff in dem transdermalen therapeutischen System enthalten. Darüber hinaus können die aus den Reaktionen und/oder Wechselwirkungen zwischen dem pharmakologisch aktiven Wirkstoff und der Polymermatrix und/oder darin enthaltenen Lösungsmitteln und/oder Monomeren aus der Polymermatrix entstandenen Reaktionsprodukte möglicherweise Reizungen auf der Haut hervorrufen. Aus dem Stand der Technik sind verschiedene Ansätze zur Erhöhung der Menge an verfügbarem Wirkstoff bekannt:
Die DE 10 141 652 A1 offenbart ein transdermales therapeutisches System mit einer Polymermatrix auf Basis von Polyacrylat-Haftklebern und beschäftigt sich mit dem Problem der Verfügbarkeit des pharmakologisch aktiven Wirkstoffs. Die Druckschrift lehrt, dass ein geringer Anteil von Monomeren, die Hydroxyl- und/oder Carboxylgruppen aufweisen, in der Polymermatrix die Verfügbarkeit des Wirkstoffs erhöht.

Die EP 2 158 905 offenbart ein transdermales therapeutisches System, das eine ausreichende Abgabe des pharmakologisch aktiven Wirkstoffs an die Haut sicherstellen soll. Die darin enthaltende Haftklebemasse basiert auf Polyacrylat. Diese Druckschrift bezieht sich jedoch lediglich auf Fentanyl als Wirkstoff.

Die WO95/18603A1 offenbart dieVerwendung von Polyvinylpyrrolidon in TTS enthaltend Polyacrylatkleber. In Beispiel 1 wird eine TTS Formulierung enthaltend Östradiol in einem Polyacrylatkleber versetzt mit PIB und PVP, Ölsäure und Dipropylenglykol offenbart. Lösungsmittel können solche mit oder ohne Carbonsäureester umfassen.

Die vorgenannten Lösungen sind nicht immer zufriedenstellend. So hat sich bei Arbeiten, die zu der vorliegenden Erfindung geführt haben herausgestellt, dass es zum Teil zu unerwünschten Wechselwirkungen zwischen dem Haftklebstoff und bestimmten pharmakologisch aktiven Wirkstoffen kommt, sodass weniger als die eigentlich zu erwartende Abgabemenge an Wirkstoff bei der Anwendung beobachtet wird. Zudem kommt es teilweise zu chemischen Abbauerscheinungen des Wirkstoffs bei Anwesenheit bestimmter Verbindungen in der Klebstoffformulierung.

Aufgabe der vorliegenden Erfindung ist es, ein Verfahren zur Verfügung zu stellen, mit dem eine wirkstoffhaltige Klebstoffformulierung zur Verwendung in einem transdermalen therapeutischen System erhalten wird, in der ein größerer Teil der während des Herstellungsprozesses eingebrachten Menge an Wirkstoff zur Verfügung steht als bei herkömmlichen Herstellungsverfahren. Außerdem soll mit Hilfe des Verfahrens eine wirkstoffhaltige Klebstoffformulierung erhalten werden, die weniger Reaktionsprodukte aufgrund von Wechselwirkungen zwischen dem pharmakologisch aktiven Wirkstoff und der Polymermatrix und/oder darin enthaltenen Lösungsmitteln aufweist. Darüber hinaus soll das Verfahren die Lagerbeständigkeit der wirkstoffhaltigen Klebstoffformulierung erhöhen. Vorzugsweise sollen die transdermalen therapeutischen Systeme über einen längeren Zeitraum, mindestens jedoch 7 Tage, auf der Haut verbleiben (können).

Diese Aufgabe wurde gelöst durch ein Verfahren zur Herstellung einer wirkstoffhaltigen Klebstoffformulierung zur Verwendung in einem transdermalen therapeutischen System umfassend die folgenden Schritte:
a) Bereitstellen einer Klebstoffformulierung enthaltend ein Carbonsäureester-basiertes Lösungsmittel und eine haftklebrige Polymermatrix, welche Monomere der Verbindungen enthält, auf denen die Polymermatrix basiert,
b) Entfernen des Carbonsäureester-basierten Lösungsmittels und der Monomere aus der Klebstoffformulierung,
c) Wiederauflösen der in Schritt b) erhaltenen Masse in einem organischen Lösungsmittel, das keine Estergruppen enthält, und Hinzufügen eines pharmakologisch aktiven Wirkstoffs, der mindestens eine Hydroxylgruppe, Carboxylgruppe, Aminogruppe und/oder Estergruppe aufweist, so dass die wirkstoffhaltige Klebstoffformulierung erhalten wird.

Der Erfindung liegt die Erkenntnis zugrunde, dass durch den Austausch des üblicherweise in für Medizinanwendungen marktüblichen Klebstoffsystemen verwendeten Carbonsäureester-basierten Lösungsmitteln durch eines ohne Estergruppen Wechselwirkungen mit dem pharmakologisch aktiven Wirkstoff reduziert oder gar unterbunden werden können, sodass eine größere und vor allem besser kalkulierbare Wirkstoffmenge tatsächlich für die Aufnahme über die Haut zur Verfügung steht. Diese Wechselwirkungen lassen sich möglicherweise - ohne an diese Theorie gebunden zu sein - mit (Veresterungs-)Reaktionen mit Hydroxyl-, Carboxyl- oder Aminogruppen beziehungsweise Umesterungsreaktionen mit Estergruppen des pharmakologisch aktiven Wirkstoffs erklären.

Nach einer bevorzugten Ausführungsform des erfindungsgemäßen Verfahrens ist das Carbonsäureester-basierte Lösungsmittel ausgewählt aus Methylacetat, Ethylacetat oder Butylacetat, wobei das Carbonsäureester-basierte Lösungsmittel bevorzugt Ethylacetat ist.

Die Restgehalte des Carbonsäureester-basierten Lösungsmittels und/oder der Monomere nach diesem Schritt b) können unabhängig voneinander jeweils < 1,0 Gew.-% betragen, bezogen auf die in Schritt b) erhaltene Masse, bevorzugt < 0,5 Gew.-%. Bevorzugt liegt der Restgehalt des Carbonsäureester-basierten Lösungsmittels im Bereich von 0,01 bis 1,0 Gew.-%, weiter bevorzugt von 0,1 bis 1,0 Gew.-%.

Bei dem erfindungsgemäßen Verfahren ist das organische Lösungsmittel vorzugsweise Tetrahydrofuran, Methanol, Isopropanol, n-Heptan, Hexan, Toluol, Methylethylketon oder Ethanol. Diese Lösungsmittel sind typischerweise gegenüber den pharmakologisch aktiven Wirkstoffen inert und eignen sich gleichzeitig für die Herstellung der Klebstoffbeschichtungen, da sie die üblichen Klebemittelmatrices lösen können und zudem ausreichend flüchtig für die anschließende Abtrennung sind.

Als Polymermatrix können im Prinzip sämtliche dem Fachmann für die Herstellung von TTS bekannte Typen ausgewählt werden. Diese sind vorzugsweise ausgewählt aus Acrylaten, Silikon-Haftklebstoffen, Polyisobutylen, SIS-Copolymeren, Silicon-Acrylat-Hybrid-Systemen, oder Mischungen davon, wobei die Polymermatrix bevorzugt ein Copolymer aus 2-Ethylhexylacrylat, Glycidylmethacrylat, 2-Hydroxyethylacrylat und/oder Vinylacetat ist. Besonders bevorzugt weist die Polymermatrix Seitenketten mit Hydroxyl- und/oder Carboxylgruppen auf.

Der pharmakologisch aktive Wirkstoff ist bevorzugt ausgewählt aus Fingolimod, Ozanimod, Teriflunomid, Baclofen oder Cladribinin, wobei der pharmakologisch aktive Wirkstoff bevorzugt Fingolimod ist. Diese Wirkstoffe zeigen typischerweise erhöhte Wechselwirkungen mit den handelsüblichen Ethylacetat-basierten Klebstoffen und sind deshalb in dem erfindungsgemäßen Verfahren bevorzugt.

Im Rahmen des erfindungsgemäßen Verfahrens kann ferner vorgesehen sein, dass in Schritt c) wenigstens ein Additiv hinzugefügt wird, wobei als Additiv bevorzugt ein Lösungsvermittler für den Wirkstoff in der Polymermatrix, ein Penetrationsbeschleuniger, ein Antioxidants, ein Weichmacher, ein Klebkraftverbesserer, Vernetzer oder Mischungen von diesen eingesetzt werden. Als Lösungsvermittler für den Wirkstoff wird vorzugsweise ein von der Polymermatrix verschiedenes Polymer eingesetzt, bevorzugt Polyvinylpyrrolidon.

Ein weiterer Gegenstand der vorliegenden Erfindung betrifft ein transdermales therapeutisches System (TTS), wie durch die Ansprüche definiert, umfassend
- einen Träger, der mit einer wirkstoffhaltigen Klebstoffformulierung hergestellt nach dem erfindungsgemäßen Verfahren weitestgehend vollflächig beschichtet ist,
- optional eine Schutzfolie, die nach Entfernen des organischen Lösungsmittels aus der Schicht der wirkstoffhaltigen Klebstoffformulierung auf dieser Schicht weitestgehend vollflächig aufgebracht ist.

Die Erfindung betrifft des Weiteren ein Verfahren zur Herstellung eines transdermalen therapeutischen Systems, umfassend die Schritte
a) Aufbringen einer wirkstoffhaltigen Klebstoffformulierung, hergestellt nach dem erfindungsgemäßen Verfahren, auf einen Träger, so dass eine den Träger weitestgehend vollflächig bedeckende Schicht erhalten wird,
b) Entfernen des organischen Lösungsmittels,
c) optional Aufbringen einer Schutzfolie auf der dem Träger abgewandten Seite der in Schritt a) aufgebrachten Schicht, wobei die Schutzfolie die Schicht weitestgehend vollflächig bedeckt.

Die Erfindung betrifft zudem das erfindungsgemäße transdermale therapeutische System zur Verwendung zur Behandlung des Hypogonadismus, zur Hormonsubstitutionstherapie, zur Behandlung von Alzheimer, von Parkinson, von Multipler Sklerose, von bipolaren Störungen, von Muskelverspannungen, von Schmerzen, von Bluthochdruck oder zur Kontrazeption, wobei die Verwendung eines transdermalen therapeutischen Systems enthaltend Fingolimod als pharmakologisch aktivem Wirkstoff zur Behandlung von Multipler Sklerose oder von Krankheiten, die durch Immunsuppression therapiert werden, bevorzugt ist.

Ein weiterer Gegenstand der vorliegenden Erfindung ist ein erfindungsgemäßes Transdermales therapeutisches System zur Verwendung in einem Verfahren zur Behandlung des Hypogonadismus, zur Hormonsubstitutionstherapie, zur Behandlung von Alzheimer, von Parkinson, von Multipler Sklerose, von bipolaren Störungen, von Muskelverspannungen, von Schmerzen, von Bluthochdruck oder zur Kontrazeption, wobei ein transdermales therapeutisches System enthaltend Fingolimod als pharmakologisch aktivem Wirkstoff zur Verwendung in einem Verfahren zur Behandlung von Multipler Sklerose oder von Krankheiten, die durch Immunsuppression therapiert werden, bevorzugt ist.

Die Erfindung betrifft auch ein Kit umfassend ein erfindungsgemäßes transdermales therapeutisches System in einer Umverpackung und optional eine Gebrauchsanweisung zur Verwendung des transdermalen therapeutischen Systems.

Die Erfindung betrifft insbesondere die folgenden Ausführungsformen:
Nach einer ersten Ausführungsform betrifft die Erfindung ein Verfahren zur Herstellung einer wirkstoffhaltigen Klebstoffformulierung zur Verwendung in einem transdermalen therapeutischen System umfassend die folgenden Schritte:
a) Bereitstellen einer Klebstoffformulierung enthaltend ein Carbonsäureester-basiertes Lösungsmittel und eine haftklebrige Polymermatrix, welche Monomere der Verbindungen enthält, auf denen die Polymermatrix basiert,
b) Entfernen des Carbonsäureester-basierten Lösungsmittels und der Monomere aus der Klebstoffformulierung,
c) Wiederauflösen der in Schritt b) erhaltenen Masse in einem organischen Lösungsmittel, das keine Estergruppen enthält, und Hinzufügen eines pharmakologisch aktiven Wirkstoffs, der mindestens eine Hydroxylgruppe, Carboxylgruppe, Aminogruppe und/oder Estergruppe aufweist, so dass die wirkstoffhaltige Klebstoffformulierung erhalten wird.

Nach einer zweiten Ausführungsform betrifft die Erfindung ein Verfahren nach Ausführungsform 1, dadurch gekennzeichnet, dass das Carbonsäureester-basierte Lösungsmittel ausgewählt ist aus Methylacetat, Ethylacetat oder Butylacetat, wobei das Carbonsäureester-basierte Lösungsmittel bevorzugt Ethylacetat ist.

Nach einer dritten Ausführungsform betrifft die Erfindung ein Verfahren nach Ausführungsform 1 oder 2, dadurch gekennzeichnet, dass die Restgehalte des Carbonsäureester-basierten Lösungsmittels und/oder der Monomere nach diesem Schritt b) unabhängig voneinander jeweils < 1,0 Gew.-% betragen, bezogen auf die in Schritt b) erhaltene Masse, bevorzugt < 0,5 Gew.-%.

Nach einer vierten Ausführungsform betrifft die Erfindung ein Verfahren nach einer der vorstehenden Ausführungsformen, dadurch gekennzeichnet, dass das organische Lösungsmittel Tetrahydrofuran, Methanol, Isopropanol, n-Heptan, Hexan, Toluol, Methylethylketon oder Ethanol ist.

Nach einer fünften Ausführungsform betrifft die Erfindung ein Verfahren nach einer der vorstehenden Ausführungsformen, dadurch gekennzeichnet, dass die Polymermatrix ausgewählt ist aus Acrylaten, Silikon-Haftklebstoffen, Polyisobutylen, SIS-Copolymeren, Silicon-Acrylat-Hybrid-Systemen, oder Mischungen davon, wobei die Polymermatrix bevorzugt ein Copolymer aus 2-Ethylhexylacrylat, Glycidylmethacrylat, 2-Hydroxyethylacrylat und/oder Vinylacetat ist.

Nach einer sechsten Ausführungsform betrifft die Erfindung ein Verfahren nach Ausführungsform 5, dadurch gekennzeichnet, dass die Polymermatrix Seitenketten mit Hydroxyl- und/oder Carboxylgruppen aufweist.

Nach einer siebten Ausführungsform betrifft die Erfindung ein Verfahren nach einer der vorstehenden Ausführungsformen, dadurch gekennzeichnet, dass der pharmakologisch aktive Wirkstoff ausgewählt ist aus Fingolimod, Ozanimod, Teriflunomid, Baclofen oder Cladribinin, wobei der pharmakologisch aktive Wirkstoff bevorzugt Fingolimod ist.

Nach einer achten Ausführungsform betrifft die Erfindung ein Verfahren nach einer der vorstehenden Ausführungsformen, dadurch gekennzeichnet, dass in Schritt c) wenigstens ein Additiv hinzugefügt wird, wobei als Additiv bevorzugt ein Lösungsvermittler für den Wirkstoff in der Polymermatrix, ein Penetrationsbeschleuniger, ein Antioxidants, ein Weichmacher, ein Klebkraftverbesserer, Vernetzer oder Mischungen von diesen eingesetzt werden.

Nach einer neunten Ausführungsform betrifft die Erfindung ein Verfahren nach Ausführungsform 8, dadurch gekennzeichnet, dass der Lösungsvermittler für den Wirkstoff ein von der Polymermatrix verschiedenes Polymer, bevorzugt Polyvinylpyrrolidon, ist.

Nach einer zehnten Ausführungsform betrifft die Erfindung ein Transdermales therapeutisches System umfassend
- einen Träger, der mit einer wirkstoffhaltigen Klebstoffformulierung hergestellt nach einem Verfahren nach einer der Ausführungsformen 1 bis 9 weitestgehend vollflächig beschichtet ist,
- optional eine Schutzfolie, die nach Entfernen des organischen Lösungsmittels aus der Schicht der wirkstoffhaltigen Klebstoffformulierung auf dieser Schicht weitestgehend vollflächig aufgebracht ist.

Nach einer elften Ausführungsform betrifft die Erfindung ein Verfahren zur Herstellung eines transdermalen therapeutischen Systems, umfassend die Schritte
a) Aufbringen einer wirkstoffhaltigen Klebstoffformulierung hergestellt nach einem Verfahren nach einer der Ausführungsformen 1 bis 9 auf einen Träger, so dass eine den Träger weitestgehend vollflächig bedeckende Schicht erhalten wird,
b) Entfernen des organischen Lösungsmittels,
c) optional Aufbringen einer Schutzfolie auf der dem Träger abgewandten Seite der in Schritt a) aufgebrachten Schicht, wobei die Schutzfolie die Schicht weitestgehend vollflächig bedeckt.

Nach einer zwölften Ausführungsform betrifft die Erfindung ein transdermales therapeutisches System nach Ausführungsform 10 zur Verwendung zur Behandlung des Hypogonadismus, zur Hormonsubstitutionstherapie, zur Behandlung von Alzheimer, von Parkinson, von Multipler Sklerose, von bipolaren Störungen, von Muskelverspannungen, von Schmerzen, von Bluthochdruck oder zur Kontrazeption, wobei die Verwendung eines transdermalen therapeutischen Systems enthaltend Fingolimod als pharmakologisch aktivem Wirkstoff zur Behandlung von Multipler Sklerose oder von Krankheiten, die durch Immunsuppression therapiert werden, bevorzugt ist.

Nach einer dreizehnten Ausführungsform betrifft die Erfindung ein Transdermales therapeutischen System nach Ausführungsform 10 zur Verwendung in einem Verfahren zur der Behandlung des Hypogonadismus, zur Hormonsubstitutionstherapie, zur Behandlung von Alzheimer, von Parkinson, von Multipler Sklerose, von bipolaren Störungen, von Muskelverspannungen, von Schmerzen, von Bluthochdruck oder zur Kontrazeption, wobei ein transdermales therapeutisches System enthaltend Fingolimod als pharmakologisch aktivem Wirkstoff zur Verwendung in einem Verfahren zur Behandlung von Multipler Sklerose oder von Krankheiten, die durch Immunsuppression therapiert werden, bevorzugt ist.

Nach einer vierzehnten Ausführungsform betrifft die Erfindung ein Kit umfassend ein transdermales therapeutisches System nach Ausführungsform 10 in einer Umverpackung und optional eine Gebrauchsanweisung zur Verwendung des transdermalen therapeutischen Systems nach Ausführungsform 12 oder 13.

### Beispiele

Die vorliegende Erfindung wird anhand der nachfolgenden Beispiele weiter erläutert, ohne jedoch darauf beschränkt zu sein.

Es wurden verschiedene Klebstoffformulierungen hergestellt, wobei unterschiedliche kommerziell erhältliche Haftklebstoffe verwendet wurden.

**Tabelle 1: Verwendete kommerziell erhältliche Haftklebstoffe**

| Haftklebstoff Nr. | Handelsname | Polymer enthaltend als Monomere | Funktion. Gruppen | Enthält Vinylacetat | Lösungsmittel im Haftklebstoff |
|---|---|---|---|---|---|
| 1 | Durotak 387-2287 | 2-Ethylhexylacrylat, Vinylacrylat | OH-Gruppen | Ja | Ethylacetat |
| | | 2-Hydroxyethylacrylat Glycidylmethacrylat | | | |
| 2 | Durotak 87-4287 | 2-Ethylhexylacrylat, Vinylacrylat | OH-Gruppen | Ja | Ethylacetat |
| | | 2-Hydroxyethylacrylat | | | |

Die Haftklebstoffe Nr. 1 und 2 sind für TTS geeignet, die mindestens sieben Tage auf der Haut getragen werden.

### Herstellung der erfindungsgemäßen Klebstoffformulierungen

Die vorgenannten kommerziell erhältlichen Haftklebstoffe wurden auf silikonisierte Folien ausgestrichen und die erhaltenen Laminate 15 Minuten bei Raumtemperatur und danach 10 Minuten bei 60 °C getrocknet. Nach der Trocknung wurden die getrockneten Haftklebestoffe von den Folien abgezogen und in einem organischen Lösungsmittel, das keine Estergruppen enthält wieder gelöst und 3 Gew.-% bezogen auf die Gesamtmasse des getrockneten Haftklebestoffs pharmakologisch aktiver Wirkstoff, sowie ggf. 10 Gew.-% Polyvinylpyrrolidon hinzugegeben. Nach einem Homogenisieren der so erhaltenen Massen wurde diese auf silikonisierte Folien ausgestrichen, so dass ein Laminat erhalten wurde. Die Laminate wurden 15 Minuten bei Raumtemperatur und danach 10 Minuten bei 60 °C getrocknet, so dass eine Klebstoffformulierung erhalten wurde. Die jeweilige Zusammensetzung der erfindungsgemäßen TTS ist in Tabelle 2 angegeben.

**Tabelle 2: Zusammensetzung der erfindungsgemäßen Klebstoffformulierungen**

| Erfindungsgem. Bsp. | Haftklebstoff Nr. | Pharm. akt. Wirkstoff | Polyvinylpyrrolidon | Lösungsmittel für die Wiederauflösung nach Trocknung |
|---|---|---|---|---|
| 1 | 1 | Fingolimod | Nein | Ethanol |
| 2 | 1 | Fingolimod | Ja | Tetrahydrofuran |
| 3 | 2 | Fingolimod | Ja | Tetrahydrofuran |

**Tabelle 3: Visuelle Beurteilung der erfindungsgemäßen Zusammensetzungen nach Hinzugabe des Wirkstoff und zweiter Trocknung**

| Erfindungsgem. Bsp. | Aussehen der Masse vor Ausstreichen | Viskosität | Aussehen des Laminats |
|---|---|---|---|
| 1 | homogen, trüb, milchig | hochviskos | homogen, trüb |
| 2 | homogen, trüb | mittelviskos | Kristalle erkennbar, trüb |
| 3 | homogen, trüb | hochviskos | Kristalle erkennbar, trüb |

### Herstellung der Referenzbeispiele

Zu den kommerziell erhältlichen Haftklebestoffen wurden jeweils 3 Gew.-%, bezogen auf die Gesamtmasse des Haftklebestoffs, pharmakologisch aktiver Wirkstoff, sowie teilweise 10 Gew.- % Polyvinylpyrrolidon hinzugegeben. Nach einem Homogenisieren der so erhaltenen Massen wurde diese auf silikonisierte Folien ausgestrichen, so dass ein Laminat erhalten wurde. Die Laminate wurden 15 Minuten bei Raumtemperatur und danach 10 Minuten bei 60 °C getrocknet, so dass eine Klebstoffformulierung erhalten wurde.

**Tabelle 4: Zusammensetzung der Referenzbeispiele**

| Referenzbsp. | Haftklebstoff Nr. | Pharm. akt. Wirkstoff | Polyvinylpyrrolidon |
|---|---|---|---|
| 2a | 1 | Fingolimod | Nein |
| 3a | 1 | Fingolimod | Ja |
| 4a | 2 | Fingolimod | Ja |

### Untersuchung der erhaltenen Klebstoffformulierungen

Der Anteil an pharmakologisch wirksamem Wirkstoff in den erhaltenen Laminaten wurde mittels angesäuertem Methanol (1 vol.-% conc. Salpetersäure in Methanol) extrahiert. Die Extrakte wurden mit einem Phosphatpuffer pH 2,5 verdünnt und anschließend mittels HPLC-Gradientenelution an einer C8-Phase und Dioden Array Detektion bei 220 nm analysiert. Als Eluenten wurden folgende Gemische verwendet:
Eluent A: 0,01-M KH₂PO₄: Acetonitril (80:20, v/v), 0,1 % Triethylamin, pH 2,5
Eluent B: 0,01-M KH₂PO₄: Acetonitril (20:80, v/v), 0,05 % Triethylamin, pH 2,5
Gradientenprofil: t[min]/B[%]: 0/35, 6/37, 8/40, 12/55, 18/100, 22/100, 24/35, 28/35
Die Quantifizierung erfolgte an einer Kalibrierkurve, die mit Fingolimod Referenzsubstanz (Externer Standard) erstellt wurde.

In der folgenden Tabelle 5 ist der Anteil an Fingolimod und evtl. Abbau- oder Nebenprodukte des Fingolimod angegeben, der in den Klebstoffformulierungen gefunden wurde. Als Nebenprodukte wurden Abbau- und Nebenprodukte des Wirkstoffs, deren UV-Spektrum demjenigen des Fingolimods gleicht, angegeben. Zur Identifizierung der Nebenprodukte wurden mittels LC/MS und LCMS/MS an 1:1-Mischungen von Fingolimods mit Ethylacetat bzw. mit Vinylacetat in Methanol gelöst und Strukturaufklärungen durchgeführt, die zu folgenden Nebenprodukten geführt haben:
- Acetamid des Fingolimods bzw. Acetyl-Fingolimod (beide haben die gleiche molare Masse von M=350, weshalb nicht bekannt ist, welches der beiden Reaktionsprodukte vorliegt),
- Vinyl-Fingolimod, M=334, sowie,
- Reaktionsprodukt von Fingolimod mit 2 Vinylgruppen, M=360.

Die Prozentangaben > 100 % beruhen zum einen auf einer Messungenauigkeit von ± 2 % und zum anderen darauf, dass bei der Herstellung der Klebstoffformulierungen im Labormaßstab Ungenauigkeiten von 1-2% hinsichtlich der Menge an Fingolimod auftreten können.

**Tabelle 5: Anteil an Fingolimod und evtl. Abbau- oder Nebenprodukte des Fingolimod in den erhaltenen Klebstoffformulierungen**

| | Anteil Fingolimod [%] | Nebenprodukte des Fingolimod [%] |
|---|---|---|
| Erfindungsgem. 1 | 102 | 0,3 |
| Referenz 1a | 98 | 2,0 |
| Erfindungsgem. 2 | 99 | 0,7 |
| Referenz 2a | 98 | 2,0 |
| Erfindungsgem. 3 | 103 | 0,4 |
| Referenz 3a | 97 | 1,2 |

| | | |
|---|---|---|
| [%] bez. auf Menge an hinzugegebenem Fingolimod | | |

Die erfindungsgemäßen Klebstoffformulierungen wiesen einen höheren Anteil an Fingolimod auf, als denjenigen der Referenzbeispiele. Darüber hinaus ist der Anteil an Nebenprodukten, die auf Reaktionen des Fingolimods mit Lösungsmitteln oder Restmonomeren aus den kommerziell erhältlichen Haftklebestoffen resultieren, bei den erfindungsgemäßen Klebstoffformulierungen deutlich reduziert. Anhand der Ergebnisse ist also ersichtlich, dass das Entfernen der Restmonomere, hier Vinylacetat, und des Lösungsmittels, hier Ethylacetat, aus den kommerziell erhältlichen Klebemassen dazu führt, dass diese Stoffe nicht mehr als Reaktionspartner für den Wirkstoff, hier Fingolimod, zur Verfügung stehen und daher eine höhere Wiederfindungsrate des Wirkstoffs in den Klebstoffformulierungen sowie ein geringerer Anteil an Nebenprodukten nachweisbar ist. Die Ergebnisse zeigen folglich, dass bei den erfindungsgemäßen Klebstoffformulierungen die Verfügbarkeit des Wirkstoffs höher ist als bei kommerziell erhältlichen, bekannten Haftklebestoffen.

### Alterungsverhalten von Haftklebestoffen enthaltend Ethylacetat und Vinylacetat

Um darzulegen, dass die in den kommerziell erhältlichen Haftklebestoffen enthaltenen Restmonomere und Lösungsmittel mit dem Wirkstoff Fingolimod reagieren und zu einer geringeren Verfügbarkeit des Wirkstoffs nach einer Lagerung beitragen, wurde mittels LC/MS und LCMS/MS an 1:1-Mischungen von Fingolimods mit Ethylacetat, bzw. mit Vinylacetat, in Methanol gelöst und die Bildung von Reaktionsprodukten von Fingolimod mit Ethylacetat und Vinylacetat, sowie Zersetzungsprodukten des Fingolimods untersucht. Die Lagerung erfolgte unter Laborbedingungen bei Raumtemperatur.

**Tabelle 6: Reaktionsprodukte des Wirkstoffs mit Restmonomeren oder Lösungsmittel nach Lagerung**

| Fingolimod mit | Alter der Probe | Anzahl der Nebenprodukte, deren Anteil > 0,2 % in der Lösung betrug | Summe aller Nebenprodukte |
|---|---|---|---|
| Vinylacetat | 2 Tage | 8 | ca. 57 % |
| Vinylacetat | 1 Monat | 16 | ca. 44 % |
| Ethylacetat | 2 Tage | 3 | ca. 15 % |

Bereits 2 Tage nach der Herstellung war eine Vielzahl von Zersetzungs- und Reaktionsprodukten des Fingolimods mit den Verbindungen erkennbar, die als Restmonomere oder Lösungsmittel in kommerziell erhältlichen Haftklebestoffen enthalten sind. Insbesondere war die Konzentration mehrerer Reaktionsprodukte mit > 0,2 % sehr hoch.

Die Untersuchungsergebnisse der Fingolimod / Vinylacetatlösung zeigen, dass sich nach 1 Monat weitere Nebenprodukte bilden, wobei die Gesamtkonzentration an Nebenprodukten jedoch nicht steigt. Bei der Interpretation der Versuchsergebnisse nach einem Monat Lagerung ist jedoch zu beachten, dass wahrscheinlich nicht alle Verbindungen nachgewiesen werden konnten, weil ihre Struktur möglicherweise dem HPLC-FLD-Detektor nicht bekannt ist.

## Patentansprüche

1. Verfahren zur Herstellung einer wirkstoffhaltigen Klebstoffformulierung zur Verwendung in einem transdermalen therapeutischen System umfassend die folgenden Schritte:
a) Bereitstellen einer Klebstoffformulierung enthaltend ein Carbonsäureester-basiertes Lösungsmittel und eine haftklebrige Polymermatrix, welche Monomere der Verbindungen enthält, auf denen die Polymermatrix basiert,
b) Entfernen des Carbonsäureester-basierten Lösungsmittels und der Monomere aus der Klebstoffformulierung,
c) Wiederauflösen der in Schritt b) erhaltenen Masse in einem organischen Lösungsmittel, das keine Estergruppen enthält, und Hinzufügen eines pharmakologisch aktiven Wirkstoffs, der mindestens eine Hydroxylgruppe, Carboxylgruppe, Aminogruppe und/oder Estergruppe aufweist, so dass die wirkstoffhaltige Klebstoffformulierung erhalten wird.

2. Verfahren nach Anspruch 1, **dadurch gekennzeichnet, dass** das Carbonsäureester-basierte Lösungsmittel ausgewählt ist aus Methylacetat, Ethylacetat oder Butylacetat, wobei das Carbonsäureester-basierte Lösungsmittel bevorzugt Ethylacetat ist.

3. Verfahren nach Anspruch 1 oder 2, **dadurch gekennzeichnet, dass** die Restgehalte des Carbonsäureester-basierten Lösungsmittels und/oder der Monomere nach diesem Schritt b) unabhängig voneinander jeweils < 1,0 Gew.-% betragen, bezogen auf die in Schritt b) erhaltene Masse, bevorzugt < 0,5 Gew.-%.

4. Verfahren nach einem der vorstehenden Ansprüche, **dadurch gekennzeichnet, dass** das organische Lösungsmittel Tetrahydrofuran, Methanol, Isopropanol, n-Heptan, Hexan, Toluol, Methylethylketon oder Ethanol ist.

5. Verfahren nach einem der vorstehenden Ansprüche, **dadurch gekennzeichnet, dass** die Polymermatrix ausgewählt ist aus Acrylaten, Silikon-Haftklebstoffen, Polyisobutylen, SIS-Copolymeren, Silicon-Acrylat-Hybrid-Systemen, oder Mischungen davon, wobei die Polymermatrix bevorzugt ein Copolymer aus 2-Ethylhexylacrylat, Glycidylmethacrylat, 2-Hydroxyethylacrylat und/oder Vinylacetat ist.

6. Verfahren nach Anspruch 5, **dadurch gekennzeichnet, dass** die Polymermatrix Seitenketten mit Hydroxyl- und/oder Carboxylgruppen aufweist.

7. Verfahren nach einem der vorstehenden Ansprüche, **dadurch gekennzeichnet, dass** der pharmakologisch aktive Wirkstoff ausgewählt ist aus Fingolimod, Ozanimod, Teriflunomid, Baclofen oder Cladribinin, wobei der pharmakologisch aktive Wirkstoff bevorzugt Fingolimod ist.

8. Verfahren nach einem der vorstehenden Ansprüche, **dadurch gekennzeichnet, dass** in Schritt c) wenigstens ein Additiv hinzugefügt wird, wobei als Additiv bevorzugt ein Lösungsvermittler für den Wirkstoff in der Polymermatrix, ein Penetrationsbeschleuniger, ein Antioxidants, ein Weichmacher, ein Klebkraftverbesserer, Vernetzer oder Mischungen von diesen eingesetzt werden.

9. Verfahren nach Anspruch 8, **dadurch gekennzeichnet, dass** der Lösungsvermittler für den Wirkstoff ein von der Polymermatrix verschiedenes Polymer, bevorzugt Polyvinylpyrrolidon, ist.

10. Transdermales therapeutisches System umfassend
- einen Träger, der mit einer wirkstoffhaltigen Klebstoffformulierung hergestellt nach einem Verfahren nach einem der Ansprüche 1 bis 9 weitestgehend vollflächig beschichtet ist, wobei der Restgehalt des Carbonsäureester-basierten Lösungsmittels nach Schritt b) 0,01 bis 1,0 Gew.-% beträgt,
- optional eine Schutzfolie, die nach Entfernen des organischen Lösungsmittels aus der Schicht der wirkstoffhaltigen Klebstoffformulierung auf dieser Schicht weitestgehend vollflächig aufgebracht ist.

11. Verfahren zur Herstellung eines transdermalen therapeutischen Systems, umfassend die Schritte
a) Aufbringen einer wirkstoffhaltigen Klebstoffformulierung hergestellt nach einem Verfahren nach einem der Ansprüche 1 bis 9 auf einen Träger, so dass eine den Träger weitestgehend vollflächig bedeckende Schicht erhalten wird,
b) Entfernen des organischen Lösungsmittels,
c) optional Aufbringen einer Schutzfolie auf der dem Träger abgewandten Seite der in Schritt a) aufgebrachten Schicht, wobei die Schutzfolie die Schicht weitestgehend vollflächig bedeckt.

12. Transdermales therapeutisches System nach Anspruch 10 zur Verwendung in der Behandlung des Hypogonadismus, zur Hormonsubstitutionstherapie, zur Behandlung von Alzheimer, von Parkinson, von Multipler Sklerose, von bipolaren Störungen, von Muskelverspannungen, von Schmerzen, von Bluthochdruck oder zur Kontrazeption, wobei die Verwendung eines transdermalen therapeutischen Systems enthaltend Fingolimod als pharmakologisch aktivem Wirkstoff zur Behandlung von Multipler Sklerose oder von Krankheiten, die durch Immunsuppression therapiert werden, bevorzugt ist.

13. Transdermales therapeutischen System nach Anspruch 10 zur Verwendung in einem Verfahren zu der Behandlung des Hypogonadismus, zur Hormonsubstitutionstherapie, zur Behandlung von Alzheimer, von Parkinson, von Multipler Sklerose, von bipolaren Störungen, von Muskelverspannungen, von Schmerzen, von Bluthochdruck oder zur Kontrazeption, wobei ein transdermales therapeutisches System enthaltend Fingolimod als pharmakologisch aktivem Wirkstoff zur Verwendung in einem Verfahren zur Behandlung von Multipler Sklerose oder von Krankheiten, die durch Immunsuppression therapiert werden, bevorzugt ist.

14. Kit umfassend ein transdermales therapeutisches System nach Anspruch 10 in einer Umverpackung und optional eine Gebrauchsanweisung zur Verwendung des transdermalen therapeutischen Systems nach Anspruch 12 oder 13.

## Claims

1. Process for producing an active-ingredient-containing adhesive formulation for use in a transdermal therapeutic system, comprising the following steps:
a) providing an adhesive formulation comprising a carboxylic ester-based solvent and a pressure-sensitive adhesive polymer matrix which comprises monomers of the compounds on which the polymer matrix is based,
b) removing the carboxylic ester-based solvent and the monomers from the adhesive formulation,
c) redissolving the composition obtained in step b) in an organic solvent which contains no ester groups, and adding a pharmacologically active ingredient which has at least one hydroxyl group, carboxyl group, amino group and/or ester group, to give the active-ingredient-containing adhesive formulation.

2. Process according to Claim 1, **characterized in that** the carboxylic ester-based solvent is selected from methyl acetate, ethyl acetate or butyl acetate, with the carboxylic ester-based solvent being preferably ethyl acetate.

3. Process according to Claim 1 or 2, **characterized in that** the residual contents of the carboxylic ester-based solvent and/or of the monomers after this step b) independently of one another are each < 1.0 wt%, based on the composition obtained in step b), preferably < 0.5 wt%.

4. Process according to any of the preceding claims, **characterized in that** the organic solvent is tetrahydrofuran, methanol, isopropanol, n-heptane, hexane, toluene, methyl ethyl ketone or ethanol.

5. Process according to any of the preceding claims, **characterized in that** the polymer matrix is selected from acrylates, pressure-sensitive silicone adhesives, polyisobutylene, SIS copolymers, silicone-acrylate hybrid systems, or mixtures thereof, with the polymer matrix preferably being a copolymer of 2-ethylhexyl acrylate, glycidyl methacrylate, 2-hydroxyethyl acrylate and/or vinyl acetate.

6. Process according to Claim 5, **characterized in that** the polymer matrix has side chains with hydroxyl and/or carboxyl groups.

7. Process according to any of the preceding claims, **characterized in that** the pharmacologically active ingredient is selected from fingolimod, ozanimod, teriflunomide, baclofen or cladribinine, with the pharmacologically active ingredient preferably being fingolimod.

8. Process according to any of the preceding claims, **characterized in that** at least one additive is added in step c), with the additive used preferably comprising a solubilizer for the active ingredient in the polymer matrix, a penetration enhancer, an antioxidant, a plasticizer, a bond strength enhancer, crosslinkers or mixtures of these.

9. Process according to Claim 8, **characterized in that** the solubilizer for the active ingredient is a polymer different from the polymer matrix, preferably polyvinylpyrrolidone.

10. Transdermal therapeutic system comprising
- a backing coated over very largely its full area with an active-ingredient-containing adhesive formulation produced by a process according to any of Claims 1 to 9, with the residual content of the carboxylic ester-based solvent after step b) being 0.01 to 1.0 wt%,
- optionally a protective film which is applied over very largely the full area of the layer of the active-ingredient-containing adhesive formulation following removal of the organic solvent from this layer.

11. Process for producing a transdermal therapeutic system, comprising the steps of
a) applying an active-ingredient-containing adhesive formulation produced by a process according to any of Claims 1 to 9 to a backing, to give a layer covering very largely the full area of the backing,
b) removing the organic solvent,
c) optionally applying a protective film on the side of the layer applied in step a) that is remote from the backing, with the protective film covering very largely the full area of the layer.

12. Transdermal therapeutic system according to Claim 10 for use in the treatment of hypogonadism, for hormone replacement therapy, for the treatment of Alzheimer's, of Parkinson's, of Multiple Sclerosis, of bipolar disorders, of muscle strains, of pain, of hypertension or for contraception, with the use of a transdermal therapeutic system comprising fingolimod as pharmacologically active ingredient for the treatment of Multiple Sclerosis or of diseases treated by immunosuppression being preferred.

13. Transdermal therapeutic system according to Claim 10 for use in a process for the treatment of hypogonadism, for hormone replacement therapy, for the treatment of Alzheimer's, of Parkinson's, of Multiple Sclerosis, of bipolar disorders, of muscle strains, of pain, of hypertension or for contraception, with a transdermal therapeutic system comprising fingolimod as pharmacologically active ingredient for use in a process for the treatment of Multiple Sclerosis or of diseases treated by immunosuppression being preferred.

14. Kit comprising a transdermal therapeutic system according to Claim 10 in an outer packaging and optionally usage instructions for the use of the transdermal therapeutic system according to Claim 12 or 13.

## Revendications

1. Procédé de préparation d'une formulation d'adhésif contenant un principe actif pour l'utilisation dans un système thérapeutique transdermique comprenant les étapes suivantes :
a) mise à disposition d'une formulation d'adhésif contenant un solvant à base d'ester d'acide carboxylique et une matrice de polymère autoadhésive qui contient des monomères des composés sur lesquels la matrice de polymère est basée,
b) élimination du solvant à base d'ester d'acide carboxylique et des monomères de la formulation d'adhésif,
c) redissolution de la masse obtenue dans l'étape b) dans un solvant organique qui ne contient aucun groupe ester, et ajout d'un principe pharmacologiquement actif qui présente au moins un groupe hydroxyle, un groupe carboxyle, un groupe amino et/ou un groupe ester, de sorte que la formulation d'adhésif contenant un principe actif est obtenue.

2. Procédé selon la revendication 1, **caractérisé en ce que** le solvant à base d'ester d'acide carboxylique est choisi parmi l'acétate de méthyle, l'acétate d'éthyle ou l'acétate de butyle, le solvant à base d'ester d'acide carboxylique étant préférablement l'acétate d'éthyle.

3. Procédé selon la revendication 1 ou 2, **caractérisé en ce que** les contenus résiduels du solvant à base d'ester d'acide carboxylique et/ou des monomères après l'étape b) sont indépendamment les uns des autres à chaque fois < 1,0 % en poids, par rapport à la masse obtenue dans l'étape b), préférablement < 0,5 % en poids.

4. Procédé selon l'une quelconque des revendications précédentes, **caractérisé en ce que** le solvant organique est le tétrahydrofuranne, le méthanol, l'isopropanol, le n-heptane, l'hexane, le toluène, la méthyléthylcétone ou l'éthanol.

5. Procédé selon l'une quelconque des revendications précédentes, **caractérisé en ce que** la matrice de polymère est choisie parmi des acrylates, des adhésifs autocollants de silicone, un polyisobutylène, des copolymères de SIS, des systèmes hybrides silicone-acrylate, ou des mélanges correspondants, la matrice de polymère étant préférablement un copolymère d'acrylate de 2-éthylhexyle, de méthacrylate de glycidyle, d'acrylate de 2-hydroxyéthyle et/ou d'acétate de vinyle.

6. Procédé selon la revendication 5, **caractérisé en ce que** la matrice de polymère présente des chaînes latérales dotées de groupes hydroxyle et/ou carboxyle.

7. Procédé selon l'une quelconque des revendications précédentes, **caractérisé en ce que** le principe pharmacologiquement actif est choisi parmi le fingolimod, l'ozanimod, le tériflunomide, le baclofène ou la cladribinine, le principe pharmacologiquement actif étant préférablement le fingolimod.

8. Procédé selon l'une quelconque des revendications précédentes, **caractérisé en ce que** dans l'étape c) au moins un additif est ajouté, dans lequel, en tant qu'additif, préférablement un agent favorisant la dissolution pour le principe actif dans la matrice de polymère, un accélérateur de pénétration, un antioxydant, un plastifiant, un agent d'amélioration de la force d'adhérence, un agent de réticulation ou des mélanges de ceux-ci, sont utilisés.

9. Procédé selon la revendication 8, **caractérisé en ce que** l'agent favorisant la dissolution pour le principe actif est un polymère différent de la matrice de polymère, préférablement une polyvinylpyrrolidone.

10. Système thérapeutique transdermique comprenant
- un support qui est revêtu essentiellement sur toute la surface par une formulation d'adhésif contenant un principe actif préparée selon un procédé selon l'une quelconque des revendications 1 à 9, la teneur résiduelle du solvant à base d'ester d'acide carboxylique après l'étape b) étant de 0,01 à 1,0 % en poids,
- éventuellement une feuille protectrice qui, après l'élimination du solvant organique de la couche de la formulation d'adhésif contenant un principe actif, est appliquée sur cette couche essentiellement sur toute la surface.

11. Procédé de préparation d'un système thérapeutique transdermique, comprenant les étapes
a) application d'une formulation d'adhésif contenant un principe actif préparée selon un procédé selon l'une quelconque des revendications 1 à 9 sur un support, de sorte qu'une couche recouvrant le support essentiellement sur toute la surface est obtenue,
b) élimination du solvant organique,
c) éventuellement application d'une feuille protectrice sur le côté opposé au support de la couche appliquée dans l'étape a), la feuille protectrice recouvrant la couche essentiellement sur toute la surface.

12. Système thérapeutique transdermique selon la revendication 10 pour l'utilisation dans le traitement de l'hypogonadisme, pour une thérapie de substitution hormonale, pour le traitement de la maladie d'Alzheimer, de la maladie de Parkinson, de la sclérose en plaques, de troubles bipolaires, de tensions musculaires, de douleurs, de l'hypertension ou pour la contraception, l'utilisation d'un système thérapeutique transdermique contenant du fingolimod en tant que principe pharmacologiquement actif pour le traitement de la sclérose en plaques ou de maladies qui sont traitées par immunosuppression, étant préférée.

13. Système thérapeutique transdermique selon la revendication 10 pour l'utilisation dans un procédé pour le traitement de l'hypogonadisme, pour une thérapie de substitution hormonale, pour le traitement de la maladie d'Alzheimer, de la maladie de Parkinson, de la sclérose en plaques, de troubles bipolaires, de tensions musculaires, de douleurs, de l'hypertension ou pour la contraception, un système thérapeutique transdermique contenant du fingolimod en tant que principe pharmacologiquement actif pour l'utilisation dans un procédé pour le traitement de la sclérose en plaques ou de maladies qui sont traitées par immunosuppression, étant préféré.

14. Kit comprenant un système thérapeutique transdermique selon la revendication 10 dans un emballage extérieur et éventuellement une notice d'utilisation pour l'utilisation du système thérapeutique transdermique selon la revendication 12 ou 13.
